(19) Europäisches Patentamt
European Patent Office
Office européen des brevets

(11) Publication number: **0 127 426**
**A1**

(12) **EUROPEAN PATENT APPLICATION**

(21) Application number: 84303461.2

(22) Date of filing: 22.05.84

(51) Int. Cl.³: A 61 K 47/00
A 61 K 37/02

(30) Priority: 23.05.83 JP 91239/83
23.03.84 JP 56922/84

(43) Date of publication of application:
05.12.84 Bulletin 84/49

(84) Designated Contracting States:
BE CH DE FR GB IT LI NL SE

(71) Applicant: Takeda Chemical Industries, Ltd.
27, Doshomachi 2-chome Higashi-ku
Osaka-shi Osaka, 541(JP)

(72) Inventor: Uda, Yoshiaki
2-501, 3 Nikawadanchi
Takarazuka Hyogo 665(JP)

(72) Inventor: Yamada, Masayuki
11-6, Daiwanishi 2-chome
Kawanishi Hyogo 666-01(JP)

(74) Representative: Laredo, Jack Joseph et al,
Elkington and Fife High Holborn House 52/54 High
Holborn
London, WC1V 6SH(GB)

(54) **Percutaneous pharmaceutical compositions for external use.**

(57) A percutaneous pharmaceutical composition which comprises at least a member selected from the group consisting of aliphatic monocarboxylic acids of 5 to 30 carbon atoms, aliphatic monohydric alcohols of 10 to 22 carbon atoms, aliphatic monoamides of 8 to 18 carbon atoms and aliphatic monoamines of 10 to 16 carbon atoms in combination with a physiologically active polypeptide. A low molecular weight polypeptide drug which can hardly be absorbed through the gastrointestinal tract can be caused to be absorbed into the human body by mere application to the skin without use of an injection syringe or relying on the oral route of administration, the pain of injection and side effects can be avoided and the desired pharmacological effect can be ensured over a sustained period of time.

EP 0 127 426 A1

Croydon Printing Company Ltd

Percutaneous Pharmaceutical Compositions for External Use

This invention relates to a percutaneous pharmaceutical composition containing a polypeptide compound having pharmacological activity, mainly central nervous system stimulating activity or hormonal activity. More particularly, this invention relates to an external drug which comprises a pharmaceutical composition containing a low molecular weight polypeptide drug which is hardly absorbed through the gastrointestinal tract in combination with one or more higher aliphatic compounds, said external drug being adapted to be absorbed through the skin in an optional region of the body to display its pharmacological action.

It is generally acknowledged that hydrophilic drugs having low oil/water partition coefficients are poorly absorbed from the gastrointestinal tract and low in bioavailability. Hydrophilic polypeptide drugs have heretofore been administered as injections but since administration by injection must rely on the hand of specialists and causes pain to patients, it has been desired to develop preparations which can be easily administered by methods other than injection and will ensure sufficient bioavailability. To solve the problem, studies have so far been conducted to improve the bioavailability of such drugs by oral, rectal and nasal administration but none of such preparations has proved sufficiently satisfactory in bioavailability and ease of

use.

To overcome these disadvantages, the present inventors conducted studies to develop a method of inçreasing the absorption of drugs by a route other than oral, rectal, and nasal, and investigated the method of promoting the absorption of low molecular weight polypeptide drugs from the skin. Regarding the percutaneous absorption of drugs, the horny layer of the skin acts as a barrier to permeation of drugs and what is important is to find a way to increase the membrane (horny layer) permeability so as to allow drugs to pass the barrier. An approach to this problem of enhancing the membrane permeability of the skin is the development of an absorption promoting agent which has the ability to soften the horny layer, dilate the pores, or control the surface-active property of the skin. However, even if the property of the horny layer is improved, this does not mean that all drugs may be absorbed through the skin. The absorbability of drugs varies a great deal according to the physicochemical properties of drugs and the type of the base used in the composition. Therefore, it is acknowledged that there exists no absorption promoting agent that makes all kinds of drugs absorbable through the skin. Thus, there exists an absorption promoting agent specific to each drug and we have to look for it.

The present inventors tentatively added to various polypeptide drugs a variety of compounds known to cause the horny layer to retain water, such as aliphatic lower alcohols such as sorbitol, glycerin, propylene glycol, etc. or compounds known to soften the horny layer such as salicylic acid, methyl salicylate, etc. but could not obtain any substantial improvement in the percutaneous absorption of the drugs. Therefore, the present inventors attempted a systematic classification of compounds which were apparently effective in promoting the absorption of said polypeptide drugs and conducted a series of experiments. As a result, it was discovered that a certain group

of higher aliphatic compounds are excellent absorption-promoting agents for polypeptide drugs.

While, among higher aliphatic compounds, mono-fatty acids of 10 to 20 carbon atoms and monovalent aliphatic alcohols of 10 to 20 carbon atoms are well known as excellent promotors being capable of increasing the percutaneous absorption of flufenamic acid which is a lipophilic drug, it was not known that these promotors also enhance the percutaneous absorption of hydrophilic polypeptide drugs.

This invention has been accomplished on the basis of the above finding.

This invention is therefore concerned with a percutaneous pharmaceutical composition which comprises at least one member selected from the group consisting of aliphatic monocarboxylic acids of 5 to 30 carbon atoms, aliphatic monohydric alcohols of 10 to 22 carbon atoms, aliphatic monoamides of 8 to 18 carbon atoms and aliphatic monoamines of 10 to 16 carbon atoms in combination with a physiologically active polypeptide.

The aliphatic monocarboxylic acids of 5 to 30 carbon atoms which are employed in accordance with this invention are normal-aliphatic fatty acids ranging from valeric acid which contains 5 carbon atoms through melissic acid which contains 30 carbon atoms, including saturated, unsaturated, straight and branched fatty acids. The saturated fatty acids include those having 5 to 30 carbon atoms such as valeric acid, isovaleric acid, caproic acid, enanthic acid, caprylic acid, pelargonic acid, capric acid, lauric acid, myristic acid, palmitic acid, margaric acid, stearic acid, arachidic acid, behenic acid, lignoceric acid, cerotic acid, montanic acid, melissic acid, etc. Particularly preferred are those fatty acids from caprylic acid which contains 8 carbon atoms through behenic acid which contains 22 carbon atoms. Unsaturated fatty acids include olefinic acids such as octenoic acid which contains 8 carbon atoms through arachidonic acid which contains 20 carbon atoms. Particularly preferred are laurolenic acid which contains 12 carbon atoms to arachidonic acid

which contains 20 carbon atoms.

The monohydric aliphatic alcohols of 10 to 22 carbon atoms which are employed in accordance with this invention include saturated, unsaturated and branched alcohols such as capryl alcohol which contains 10 carbon atoms through behenyl alcohol which contains 22 carbon atoms. Preferred are lauryl alcohol which has 12 carbon atoms through stearyl alcohol which contains 18 carbon atoms.

The aliphatic monoamides of 8 to 18 carbon atoms which are employed in accordance with this invention includes octylamide which contains 8 carbon atoms through stearylamide which contains 18 carbon atoms. Preferred are laurylamide which contains 12 carbon atoms through myristylamide which contains 14 carbon atoms.

The aliphatic monoamine of 10 to 16 carbon atoms which employed in accordance with this invention includes decylamine which has 10 carbon atoms through hexadecylamine which contains 16 carbon atoms. Preferred are decylamine of 10 carbon atoms through dodecylamine of 12 carbon atoms.

The effective proportion of said higher aliphatic compound (absorption promoting agent) is not less than 0.1% by weight, and the range of 1% by weight to 10% by weight is particularly desirable.

The polypeptides are exemplified by peptides which comprise 2 to 10 peptide linkages. The polypeptides have a small oil/water partition coefficient and more particularly an octanol/water partition coefficient of not more than 0.1. The oil/water partition coefficient can be determined by the method described in Robert E. Notari "Biopharmaceutics and pharmacokinetics", Marcel Dekker Inc., 1975, New York, U.S.A.

As the physiologically active polypeptide, there may be mentioned, for example, L-pyroglutamyl-L-histidyl-L-prolinamide (thyrotropin releasing hormone; hereinafter referred to briefly as TRH) or its salts, especially its tartrate [U.S. Patent No. 3,957,247], and polypeptides

represented by the formula [I]:

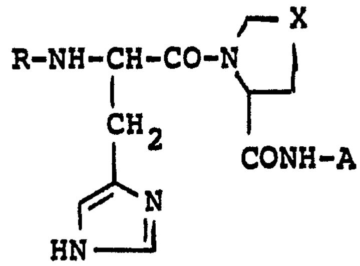

[I]

wherein A stands for hydrogen, alkyl, aralkyl, alkoxyalkyl, hydroxyalkyl or alkoxy, R stands for 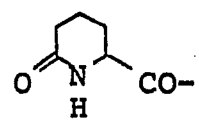 , 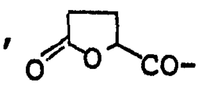 or 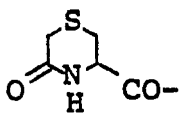 , X stands for $-CH_2-$, $-CH_2CH_2-$ or $-S-$, R and each of the other constituent aminoacid residues may have L- or D-configuration or be racemic] and salts thereof [U.S. Patent No. 4,100,152].

Among the compounds represented by the formula [I], the citrate of the compound shown below is referred to briefly as "DN-1417".

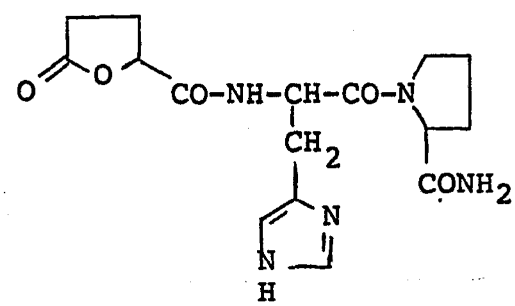

(γ-butyrolactone-γ-carbonyl-L-histidyl-L-prolinamide)

Furthermore, as the polypeptide, there may be mentioned luteinizing hormone-releasing hormones (hereinafter referred to briefly as "LH-RH") or peptides which have the LH-RH activity and have the formula (II).

(Pyr)Glu-$R_1$-Trp-Ser-$R_2$-$R_3$-$R_4$-Arg-Pro-$R_5$ (II)

[wherein $R_1$ stands for His, Tyr, Trp or p-$NH_2$-Phe; $R_2$ stands for Tyr or Phe; $R_3$ stands for Gly or a D-amino

acid residue: $R_4$ stands for Leu, Ile or Nle; $R_5$ stands for Gly-NH-$R_6$ ($R_6$ stands for H or a lower alkyl group which may optionally have a hydroxyl group) or NH-$R_6$ ($R_6$ has the meaning defined above)] [U.S. Patent No. 3,853,837, U.S. Patent No. 4,008,209, U.S. Patent No. 3,972,859. British Patent No. 1,423,083, Proceedings of the National Academy of Science of the United States of America, vol. 78, pp. 6509-6512 (1981)].

As examples of the D-aminoacid residue $R_3$, there may be mentioned the residues of alpha-D-aminoacids containing up to 9 carbon atoms (e.g. D-Leu, Ile, Nle, Val, Nval, Abu, Phe, Phg, Ser, Thr, Met, Ala, Trp, α-Aibu, etc.), which may have suitable protective groups (e.g. t-butyl, t-butoxy, t-butoxycarbonyl, etc.). Of course, salts of the peptide (II) with acids as well as metal complex compounds of the peptide (II) may also be employed just as peptide (II). All abbreviations, wherever they are used in this specification to denote aminoacids, peptides, protective groups, etc., are those according to IUPAC-IUB Commission on Biochemical Nomenclature or those commonly employed in the particular field of art. Where any of the aminoacids named herein is subject to optical isomerism, all references to such aminoacid mean the L-form unless otherwise indicated.

In the present specification, the polypeptide (II) in which $R_1$=His, $R_2$=Tyr, $R_3$=D-Leu, $R_4$=Leu, $R_5$=$NHCH_2$-$CH_3$ is referred to briefly as TAP-144.

Examples of said polypeptide include vasopressin, vasopressin derivatives {desmopressin [Folia Endocrinologica Japonica, 54, 5, pp. 676-691, (1978)]}, enkephalin, enkephalin derivatives [U.S. Patent 4,277,394; European Patent Application Publication No. 31567] and substance P.

The percutaneous pharmaceutical composition can be produced by compounding or mixing with an absorption promoting agent. The proportion of said polypeptide in the percutaneous pharmaceutical composition is generally 0.0005 to 50% by weight, and 10 μg to 50 mg per dose unit.

The base for use in the external drug preparation according to this invention includes polyalcohols such as propylene glycol, sorbitol, glycerin, polyethylene glycol, etc., begetable oils and fats such as olive oil,

safflower oil, cottonseed oil, etc., animal oils and fats such as squalene, squalane, lanolin, etc., paraffins such as liquid paraffin, vaseline, etc., fatty acid esters such as isopropyl myristate, isopropyl palmitate, diethyl sebacate, etc., and cellosolve, methyl-cellosolve, etc. While these bases can be used in an optional proportion, the preferred range is 50 to 95% based on the total weight of the external drug preparation. In addition to said absorption promoting agent and base, the percutaneous absorption external drug according to this invention may contain a component for prolonging the duration of blood concentration of the active component or a component for controlling the rate of percutaneous absorption, such as solid paraffin, beeswax, carnauba wax, hydrogenated castor oil, lanolin, polyethylene glycol (PEG-1500 and up), sperm wax, glyceryl monostearate, cholesterol, carboxypolymethylene, carboxymethylcellulose, carboxyethylcellulose, silicone resin and the like, in suitable proportions.

In accordance with this invention, such a percutaneous pharmaceutical composition containing said components can be applied to the human body surface, either as it is or as formulated into any of the hydrophilic and oleagenous ointments and emulsions. Alternatively, such pharmaceutical composition can be impregnated into, or deposited on, a suitable support material and applied to the skin in such application forms as an adhesive tape, sheet, patch or the like.

The support material mentioned just above is exemplified by high polymer film, woven fabric, non-woven fabric, paper and so forth. When the composition is made available in the form of an adhesive tape, sheet or patch, the adhesive agent may be selected from among such materials as polyalkylvinylether, polyalkylacrylate, polyisobutylene, natural rubber, synthetic rubber and other types. Further, for the purpose of imparting suitable degrees of plasticity and adhesivity, it is

possible to add animal or vegetable oil, vaselin, lanolin or the like, or/and, as an antiphlogistic component, an antihistaminic agent such as diphenhydramine, etc.

Since, in accordance with this invention, a low molecular weight polypeptide drug which can hardly be absorbed through the gastrointestinal tract can now be caused to be absorbed into the human body by mere application to the skin without use of an injection syringe or relying on the oral route of administration, the pain of injection and side-effects can be avoided and the desired pharmacological effect can be ensured over a sustained time period.

The invention is now described in further detail by means of examples and comparative examples, as follows.

Example 1

0.8 mg of TRH tartrate and 10 μCi of $^3$H-labeled TRH (specific radioactivity 100 ci/mmol) were dissolved in a mixture of 179.2 mg of propylene glycol and 20 mg of oleic acid which is an absorption promoting agent according to this invention, and the solution was used as a coating sample. SD-JCL male rats with an average body weight of 250 g were used as test animals. Under pentobarbital anesthesia, the abdomen of each animal was clipped off hairs (20 $cm^2$: 5 x 4 cm) with an electric clipper and the sample was applied to the denuded area. Using 5 rats, 0.12 ml of venous blood was taken from the tail vein 1, 2, 4 and 6 hours after application of the sample. Each blood specimen was centrifuged and 0.05 ml of the plasma was put in a polyethylene minivial containing 5 ml of a toluenic scintillator and stirred. After standing, the total radioactivity of the plasma sample was measured with a β-ray scintillation spectrometer and the TRH equivalent concentration of the plasma (μg.eq./ml) was calculated.

The percutaneous absorption of TRH tartrate was evaluated in $AUC_0^6$ (the area under the blood concentration-time curve during 0 to 6 hours). Thus, the multiple of the $AUC_0^6$ value of the preparation containing a percutaneous absorption promoting agent in accordance with this invention over the $AUC_0^6$ value of the control preparation which did not contain the absorption promoter according to Comparative Example 1 was determined. The mean $AUC_0^6$ of TRH tartrate in the preparation containing the absorption promoting agent for 5 rats was 1.3 μg.eq.hr/ml. This value was approximately 8 times as large as the $AUC_0^6$ (0.16 μg.eq.hr/ml) of the preparation of Comparative Example 1. It is therefore apparent that the percutaneous absorption of TRH tartrate was remarkably improved by the addition of oleic acid according to this invention.

Comparative Example 1

In 199.2 mg of propylene glycol were dissolved 0.8 mg of TRH tartrate and 10 μCi of $^3H$-labeled TRH (specific radioactivity 100 Ci/mmol) to prepare a coating sample.

The percutaneous absorption test and evaluation in rats were performed in the same manner as Example 1. The $AUC_0^6$ value of this preparation was as low as 0.16 μg.eq/ml, indicating that this preparation would not produce the desired pharmacological effect.

Comparative Example 2

0.8 mg of TRH tartrate and 10 μCi of $^3H$-labeled TRH (specific radioactivity 100 Ci/mmol) were dissolved in a mixture of 40 mg of isopropyl myristate, an additive anticipated to promote absorption of the drug, and 159.2 mg of propylene glycol to prepare a coating sample. The percutaneous absorption test and evaluation were conducted in the same manner as Example 1. The $AUC_0^6$ value of this preparation was as low as 0.18 μg.eq/ml, which is close to the $AUC_0^6$ of the preparation of Comparative Example 1, showing that isopropyl myristate has no absorption promoting effect.

The same experiment was also carried out using isopropyl palmitate but this additive had no absorption

promoting effect, either.

Comparative Example 3

3 mg of clonidine, an antihypertensive drug, was dissolved in a mixture of 20 mg of lauric acid, one of the percutaneous absorption promoting agents according to this invention, and 177 mg of propylene glycol to prepare a coating sample.

A percutaneous absorption test was carried out in male SD-JCL rats with an average body weight of 250 g. Under pentobarbital anesthesia, the abdomen of each rat was clipped of hairs with an electric clipper and the sample was coated on the denuded abdominal area (5 x 4 cm).

Using 5 rats, 0.5 ml of venous blood was taken from the tail vein 1, 2, 4 and 6 hours after application of the sample and the blood concentration of clonidine was determined by the following procedure.

The collected venous blood was centrifuged and 0.2 ml of the plasma was extracted with 1 ml of 0.01 N-NaOH and 5 ml of chloroform. From a 4 ml portion of the chloroform layer, the chloroform was distilled off and the residue was dissolved by addition of 0.2 ml of 0.05 M sodium acetate-acetonitrile-tetrahydrofuran (70:30:0.2, v/v). A 50 µl portion of the solution was applied to a liquid chromatograph to determine the concentration of clonidine. The µ-Bondapak C18 column was used for this liquid chromatography. The percutaneous absorption of clonidine was evaluated in terms of $AUC_0^6$ (area under the blood concentration-time curve of clonidine during 0 to 6 hours) and its multiple over the $AUC_0^6$ of the preparation of Comparative Example 1 was calculated.

As a result, clonidine could not be detected in the blood and the percutaneous absorption promoting agent lauric acid according to this invention was found to be ineffective for clonidine. The same experiment was also performed using the other percutaneous absorption promoting agents of this invention, namely oleic acid, lauryl

alcohol, oleamide and laurylamine, but they were also ineffective for clonidine.

Example 2

0.8 mg of DN-1417 and $^{14}C$-labeled DN-1417 (specific radioactivity 15.3 mCi/mmol) were dissolved in a mixture of 30 mg of lauric acid which is an absorption promoting agent according to this invention and 169.2 mg of propylene glycol to prepare a coating sample. The percutaneous absorption test and evaluation in rats were performed in the same manner as Example 1. The $AUC_0^6$ value for the preparation according to the invention was 2.6 μg.eq.hr/ml whereas the $AUC_0^6$ value for a control, namely a propylene glycol-based preparation of DN-1417 which was free of the absorption promoting agent was 0.3 μg.eq.hr/ml. In accordance with the invention, the absorption was thus 8.5 times higher as compared with the control.

Example 3

0.8 mg of TRH tartrate and 10 μCi of $^{3}H$-labeled TRH (specific radioactivity 100 Ci/mmol) were dissolved in a mixture of 179.2 mg of propylene glycol and 20 mg of lauric acid, which is one of the absorption promoting agents according to this invention, to prepare a coating sample. The percutaneous absorption test in rats was conducted in the same manner as Example 1, except that in this example the abdominal treatment site of the rat was wiped 5 times with ethanol-sanitary cotton and, then, the sample was applied. The evaluation of percutaneous absorption was made in the same manner as Example 1.

The $AUC_0^6$ value of TRH tartrate in the above preparation as used according to the procedure of this example was 2.22 μg.eq.hr/ml. This value was 14 times as high as the $AUC_0^6$ value of the control preparation according to Comparative Example 1, and the percutaneous absorption of TRH tartrate was remarkably promoted by ethanol wiping.

Example 4

0.8 mg of TRH tartrate and 10 μCi of $^3$H-labeled TRH (specific radioactivity 100 Ci/mmol) were dissolved in a mixture of 179.2 mg of propylene glycol and 20 mg of lauric acid, which is one of the absorption promoting agents according to this invention, to prepare a coating sample. The percutaneous absorption test in rats was conducted in the same manner as Example 1 except that, in this example, the horny layer of the clipped abdomen (20 $cm^2$) was stripped 3 times with a cellophane tape and, then, the sample was applied. The evaluation of percutaneous absorption was made by the same procedure as in Example 1.

The $AUC_0^6$ value of TRH tartrate in the above preparation as used according to the procedure of this example was 6.06 μg.eq.hr/ml. This value was 38 times as high as the $AUC_0^6$ value of the control preparation according to Comparative Example 1 and the percutaneous absorption was remarkably promoted.

Example 5

0.5 mg of TRH and 10 μCi of $^3$H-labeled TRH (specific radioactivity 100 Ci/mmol) were dissolved in a mixture of 169.5 mg of propylene glycol and 30 mg of oleic acid, which is one of the absorption promoting agents according to this invention to prepare a coating sample. The percutaneous absorption test and evaluation in rats were carried out in the same manner as Example 1. The $AUC_0^6$ value of this example was 1.4 μg.eq.hr/ml. This value was 8.7 times as large as the $AUC_0^6$ value of Comparative Example 1 (0.16 μg.eq.hr/ml), showing that the percutaneous absorption of TRH was remarkably enhanced by the addition of oleic acid according to this invention.

Example 6

150 μg of TAP-144 was added to a mixture of 180 mg of propylene glycol and 20 mg of lauric acid, which is one of the absorption promoting agents according to this

invention, to prepare a coating sample. The percutaneous absorption test and evaluation in rats were carried out in the same manner as Example 1, except that the determination of TAP-144 was by radioimmunoassay. The $AUC_0^6$ value of this example was 6.20 ng.hr/ml, which was 12 times as large as the $AUC_0^6$ value (0.50 ng.hr/ml) of a control preparation which did not contain an absorption promoting agent. It is thus clear that the percutaneous absorption of TAP-144 was remarkably promoted by the addition of lauric acid according to this invention.

Comparative Example 4

0.8 mg of TRH tartrate and 10 µCi of $^3$H-labeled TRH (specific radioactivity 100 Ci/mmol) were dissolved in a mixture of 179.2 mg of propylene glycol and 20 mg of propionic acid to prepare a coating sample. The percutaneous absorption test and evaluation in rats were carried out in the same manner as Example 1. The $AUC_0^6$ value of this preparation was as low as 0.18 µg.eq/ml and the amount of absorption was barely expected.

Comparative Example 5

0.8 mg of TRH tartrate and 10 µCi of $^3$H-labeled TRH (specific gravity 100 Ci/mmol) were dissolved in a mixture of 179.2 mg of propylene glycol and 20 mg of hentriacontanic acid ($C_{31}$), which was expected to promote absorption, to prepare a coating sample. The percutaneous absorption test and evaluation in rats were carried out in the same manner as Example 1. The $AUC_0^6$ value of this example was as low as 0.17 µg.eq/ml, which was too low to display its pharmacological effect at all.

Comparative Example 6

0.8 mg of TRH tartrate and 10 µCi or $^3$H-labeled TRH (specific radioactivity 100 Ci/mmol) were dissolved in a mixture of 179.2 mg of propylene glycol and 20 mg of caprylyl alcohol ($C_8$) to prepare a coating sample. The percutaneous absorption test and evaluation in rats were carried out in the same manner as in Example 1. The

$AUC_0^6$ value of this preparation was as low as 0.19 μg.eq/ml, which was too low to ensure the desired pharmacological effect.

Comparative Example 7

0.8 mg of TRH tartrate and 10 μCi of $^3H$-labeled TRH (specific radioactivity 100 Ci/mmol) were dissolved in a mixture of 179.2 mg of propylene glycol and 20 mg of carnaubyl alcohol ($C_{24}$) to prepare a coating sample. The percutaneous absorption test and evaluation in rats were conducted in the same manner as Example 1. The $AUC_0^6$ value of this preparation was as low as 0.15 μg.eq/ml, which was too low to ensure the desired pharmacological effect.

Example 7

10 mg of TRH tartrate was dissolved in a mixture of 20 mg of oleic acid and 1970 mg of propylene glycol under heating to prepare a percutaneous pharmaceutical composition.

2 g of the composition were poured into a container having a size of 6 cm x 5 cm which had been prepared by heat-sealing a polyethylene-laminated aluminum foil as a support material and a high-density polyethylene film (High pore® 2200, Asahi Chemical Industry Co., Ltd.) capable of controlling release of TRH tartrate.

The container was sealed and trimmed, and this trimmed part was coated with polyalkyl acrylate as an adhesive agent, followed by joining the coated part with a release paper to prepare a patch.

What is claimed is:

1. A percutaneous pharmaceutical composition which comprises at least a member selected from the group consisting of aliphatic monocarboxylic acids of 5 to 30 carbon atoms, aliphatic monohydric alcohols of 10 to 22 carbon atoms, aliphatic monoamides of 8 to 18 carbon atoms and aliphatic monoamines of 10 to 16 carbon atoms in combination with a physiologically active polypeptide.

2. A percutaneous pharmaceutical composition as claimed in Claim 1, wherein the physiologically active polypeptide is one having 2 to 10 peptide linkages.

3. A percutaneous pharmaceutical composition as claimed in Claim 1, wherein the polypeptide is (Pyr)Glu-His-Trp-Ser-Tyr-D-Leu-Leu-Arg-Pro-NH-$C_2H_5$.

4. A percutaneous pharmaceutical composition as claimed in Claim 1, wherein the polypeptide is L-pyroglutamyl-L-histidyl-L-prolinamide.

5. A percutaneous pharmaceutical composition as claimed in Claim 1, wherein the polypeptide is γ-butyrolactone-γ-carbonyl-L-histidyl-L-prolinamide.

6. A percutaneous pharmaceutical composition as claimed in Claim 1, wherein the aliphatic monocarboxylic acid is oleic acid.

7. A percutaneous pharmaceutical composition as claimed in Claim 1, wherein the aliphatic monocarboxylic acid is lauric acid.

8. A percutaneous pharmaceutical composition as claimed in Claim 1, wherein the proportion of the higher aliphatic

compound is not less than 0.1% by weight.

European Patent Office

# EUROPEAN SEARCH REPORT

0127426

Application number

EP 84 30 3461

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A-2 427 100 (KALI-CHEMIE PHARMA GmbH) * Page 1, line 21 - page 2, line 25; page 10, lines 1-12; claims 1-3 * | 1-8 | A 61 K 47/00<br>A 61 K 37/02 |
| E | EP-A-0 103 783 (DR. THILO & CO.) * Page 14, lines 1-16; claims 1-3 * | 1-8 | |
| P,X | EP-A-0 086 092 (SMITHKLINE BECKMAN CORP.) * Page 11, lines 1-11; claims 1-3 * | 1-8 | |
| A | GB-A-2 015 339 (SANDOZ LTD.) * Page 4, lines 46-66; claims 22-28 * | 1-8 | **TECHNICAL FIELDS SEARCHED (Int. Cl. 3)**<br>A 61 K |

The present search report has been drawn up for all claims

| Place of search | Date of completion of the search | Examiner |
|---|---|---|
| THE HAGUE | 31-08-1984 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document